# EUROPEAN PATENT SPECIFICATION

(11) **EP 1 839 568 B1**
(45) Date of publication and mention of the grant of the patent: **07.03.2018**
(21) Application number: 06711737.4
(22) Date of filing: 16.01.2006
(51) Int. Cl.: G06F 19/00, A61B 5/00, A61B 18/00, A61B 90/00

(54) **OPERATION DATA MANAGEMENT DEVICE, OPERATION CONTROL DEVICE, AND OPERATION DATA PROCESSING METHOD**
VORRICHTUNG ZUR VERWALTUNG VON OPERATIONSDATEN, OPERATIONSSTEUERUNGSVORRICHTUNG UND VERFAHREN ZUR VERARBEITUNG VON OPERATIONSDATEN
DISPOSITIF DE SUIVI DES DONNEES OPERATIONELLES, APPAREIL DE SURVEILLANCE OPERATIONELLE ET PROCEDE DE TRAITEMENT DES DONNEES OPERATIONELLES

(30) Priority: 19.01.2005 JP 2005012088
(43) Date of publication of application: 03.10.2007
(73) Proprietor: Olympus Corporation, Tokyo 192-8507 (JP)
(72) Inventor: TASHIRO, Koichi, Tokyo 151-0072 (JP); UCHIKUBO, Akinobu, Tokyo 151-0072 (JP); OZAKI, Takashi, Tokyo 151-0072 (JP); IMAMIYA, Chie, Tokyo 151-0072 (JP); NAKAMURA, Takeaki, Tokyo 151-0072 (JP); GOTANDA, Masakazu, Tokyo 151-0072 (JP)
(74) Representative: von Hellfeld, Axel
(86) International application number: PCT/JP2006/300456
(87) International publication number: WO 2006/077797

(56) References cited:
- WO-A1-02/17640
- FR-A1- 2 853 980
- JP-A- 2001 170 008
- JP-A- 2002 233 535
- JP-A- 2003 070 748
- JP-A- 2004 318 757
- JP-A- 2004 507 941
- JP-A- 2004 528 104
- US-A- 5 860 918
- US-A1- 2003 145 854

## Description

### Technical Field

The present invention relates to an operation data management device, an operation control device, and an operation data processing method, and particularly to an operation data management device, an operation control device, and an operation data processing method capable of performing efficient data recording.

### Background Art

Conventionally, various types of operation data have been recorded in an operation. The operation data includes, for example, biological data including the pulse, the blood pressure of a patient and so forth, and data of a medical device employed in the operation, such as an electric scalpel. The purpose of the recording is to prepare for, for example, the occurrence of the need for a postoperative check of the content of the operation by a surgeon in the event of a change in the condition of the patient after the operation, for example. When such a need arises, the surgeon can postoperatively check the operation content of the operation by viewing the various types of the recorded data of the operation displayed on a display device.

As a technique of recording such data of the operation, there is a technique described in Japanese Unexamined Patent Application Publication No. 2002-233535.

Even in a single operation, however, all of the operation data relating to the operation is enormous in amount. It is therefore inefficient to record data which will not need to be checked later.

In light of the above, an object of the present invention is to provide an operation data management device capable of performing data management of higher efficiency.

WO 02/17640 A1 discloses a system for monitoring the execution of a procedure. The system comprises recording devices for recording characteristics of the procedure during procedure. These characteristics can be audio/video data.

US 5,860,918 A discloses a medical monitoring device comprising a system for monitoring and recording physiological parameters of a patient. The monitored physiological parameters are compared by a comparator with a threshold associated with respective physiological parameters, depending on an operation mode preselected by a user, and stored in a memory.

FR2853980 discloses a system for recording the performance of gas insufflator during a medical operation, detecting errors and abnormalities.

### Disclosure of Invention

### Means for Solving the Problem

The invention is defined in claim 1.

An operation data management device according to a first aspect of the present invention includes a setting unit for setting operation data to be recorded out of inputted operation data, and an operation data recording unit for recording the operation data set by the setting unit in a recording device in association with a predetermined time.

An operation data management device according to a second aspect of the present invention includes a first operation data recording unit for recording inputted operation data in a first recording unit in association with a predetermined time, a first setting unit for setting operation data to be recorded in a second recording unit out of the operation data recorded in the first recording unit, and a second operation data recording unit for recording the operation data set by the first setting unit in the second recording unit in association with a predetermined time.

An operation data processing method according to a third aspect of the present invention is an operation data processing method of recording operation data and displaying or printing the recorded operation data. According to the method, the operation data including at least biological data of a patient and data outputted from a medical device is recorded in a first recording unit in association with a predetermined time, operation data to be recorded in a second recording unit is set out of the operation data recorded in the first recording unit, operation data to be displayed on a display device is set out of the operation data recorded in the first recording unit or the second recording unit, operation data to be printed by a printing device is set out of the operation data recorded in the first recording unit or the second recording unit, the operation data set to be displayed is displayed on the display device, and the operation data set to be printed is printed by the printing device.

An operation control device according to a fourth aspect of the present invention is an operation control device connected to a plurality of medical devices to be able to communicate with the medical devices. The device includes a setting unit for setting operation data to be recorded out of operation data of each of the plurality of medical devices received from the medical devices through communication, an operation data recording unit for recording the operation data set by the setting unit in a recording unit in association with a predetermined time, and a medical device control unit for controlling a predetermined one of the plurality of medical devices through the communication with the predetermined device.

### Brief Description of the Drawings

Fig. 1 is a configuration diagram illustrating a configuration of an operation system according to an embodiment.
Fig. 2 is a diagram illustrating an example of a recording target data setting screen according to the embodiment.
Fig. 3 is a diagram illustrating an example of an individual data setting screen according to the embodiment.
Fig. 4 is a diagram illustrating a configuration example of recording target set data according to the embodiment.
Fig. 5 is a diagram illustrating that data is recorded only when a recording condition is matched.
Fig. 6 is a diagram illustrating that all data is recorded.
Fig. 7 is a diagram illustrating that data is not the target of the recording and thus is not recorded at all.
Fig. 8 is a diagram illustrating an example of a confirmation screen for confirming a set content according to the embodiment.
Fig. 9 is a flowchart illustrating an example of the flow of processing of setting the recording target data according to the embodiment.
Fig. 10 is a flowchart illustrating an example of the flow of processing of recording the operation data according to the embodiment.
Fig. 11 is a diagram illustrating an example of a display target data setting screen according to the embodiment.
Fig. 12 is a diagram illustrating an example of a print target data setting screen according to the embodiment.
Fig. 13 is a graph for explaining which range of data is recorded when a predetermined threshold value has been set as the recording condition.
Fig. 14 is another graph for explaining which range of data is recorded when a predetermined threshold value has been set as the recording condition.
Fig. 15 is graphs illustrating an example in which three or more operation data are displayed on the screen of a display device, being aligned in terms of the time axis.
Fig. 16 is another type of graphs illustrating an example in which three or more operation data are displayed on the screen of a display device, being aligned in terms of the time axis.
Fig. 17 is graphs illustrating an example in which the condition for the display is set to display only data showing a simultaneous change out of the operation data.
Fig. 18 is a system configuration diagram illustrating an example in which a system controller is also connected to a medical device on which the system controller does not perform control, but with which the system controller performs data communication.
Fig. 19 is a diagram illustrating an example of graphs displaying, as the operation data of a gas insufflator, performance data of the gas insufflator recorded in association with a predetermined time.
Fig. 20 is a diagram illustrating an example of a setting screen for changing the time interval of a recording time.

### Best Mode for Carrying Out the Invention

An embodiment will be described below with reference to the drawings.

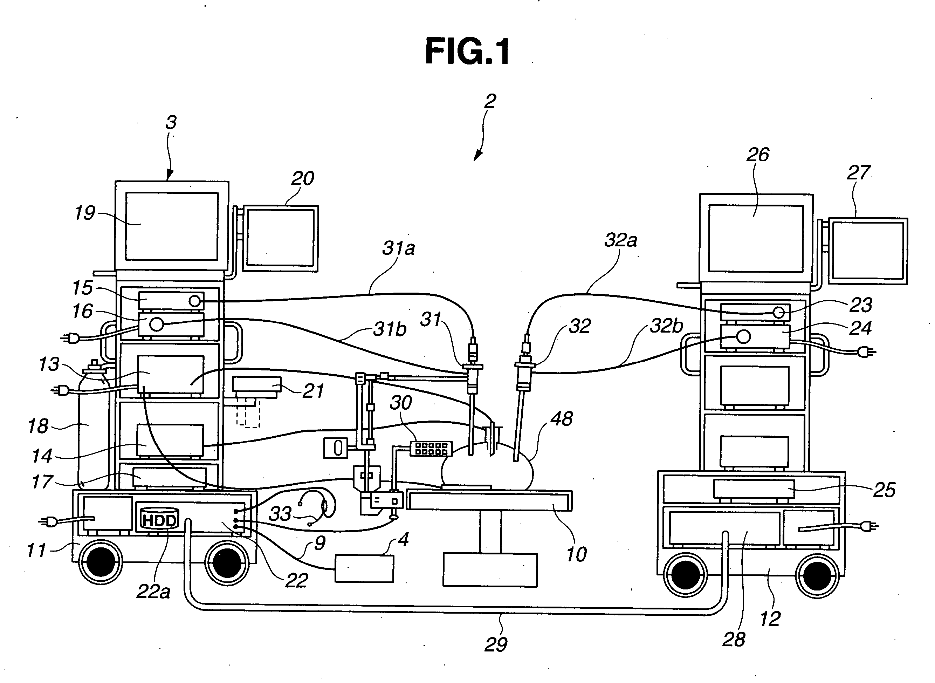

Description will be first made of an overall configuration of an endoscopic operation system, which is a medical system placed in an operating room. Fig. 1 is a configuration diagram illustrating a configuration of an operation system according to the present embodiment.

As illustrated in Fig. 1, a patient bed 10 on which a patient 48 lies and an endoscopic operation system 3 are placed in an operating room 2. The endoscopic operation system 3 includes a first cart 11 and a second cart 12.

The first cart 11 is mounted with a gas cylinder 18 filled with, for example, carbon dioxide, and devices such as an ultrasonic operation device 13, a gas insufflator 14, an endoscopic camera device 15, a light source device 16, and a video tape recorder (VTR) 17, for example, which are medical devices functioning as controlled devices. The endoscopic camera device 15 is connected to a first endoscope 31 via a camera cable 31a. The light source device 16 is connected to the first endoscope 31 via a light guide cable 31b.

The first cart 11 is further mounted with a display device 19, a first central display panel 20, an operation panel 21, and so forth. The display device 19 is, for example, a TV monitor for displaying an endoscopic image and so forth.

The first central display panel 20 forms a display device capable of selectively displaying all data obtained during an operation. The operation panel 21 is formed by a display unit such as a liquid crystal display, for example, and a touch panel integrally provided on the display unit, for example. The operation panel 21 forms a central operation device operated by a nurse or the like located in a non-sterile area.

Further, the first cart 11 is mounted with a system controller 22, which forms a control device. The system controller 22 is connected to the ultrasonic operation device 13, the gas insufflator 14, the endoscopic camera device 15, the light source device 16, and the VTR 17 described above via not-illustrated communication lines. The system controller 22 is connectable to a headset type microphone 33. Further, the system controller 22 has a sound recognition function to recognize the sound inputted from the microphone 33 so that the system controller 22 can control the respective devices in accordance with the sound of a surgeon. That is, the system controller 22 includes a central processing unit (CPU), and further includes a medical device control unit for communicating with a predetermined one of the plurality of medical devices connected thereto to control the predetermined device.

The system controller 22 includes therein a recording device for recording various types of data, which is a hard disk drive (hereinafter referred to as the HDD) 22a in the present example. As described later, the system controller 22 forms an operation data management device for recording operation data in the HDD 22a in association with the elapsed time of the operation. The operation data includes, for example, performance data, set data, and inputted data of the respective medical devices connected to the system controller 22.

Meanwhile, the second cart 12 is mounted with an endoscopic camera device 23, a light source device 24, an image processing device 25, a display device 26, and a second central display panel 27, which function as controlled devices.

The endoscopic camera device 23 is connected to a second endoscope 32 via a camera cable 32a. The light source device 24 is connected to the second endoscope 32 via a light guide cable 32b.

The display device 26 displays an endoscopic image and so forth obtained by the endoscopic camera device 23. The second central display panel 27 can selectively display all data obtained during an operation.

The endoscopic camera device 23, the light source device 24, and the image processing device 25 are connected via not-illustrated communication lines to a relay unit 28 mounted on the second cart 12. Further, the relay unit 28 is connected by a relay cable 29 to the system controller 22 mounted on the above-described first cart 11.

Accordingly, the system controller 22 performs central control of the endoscopic camera device 23, the light source device 24, and the image processing device 25, which are mounted on the second cart 12, and the ultrasonic operation device 13, the gas insuffaltor 14, the endoscopic camera device 15, the light source device 16, and the VTR 17, which are mounted on the first cart 11. Therefore, when communication is established between the system controller 22 and the above devices, the system controller 22 can display, on the liquid crystal display of the above-described operation panel 21, a setting screen displaying the setting conditions of the connected devices, operation switches, and so forth. Further, when a predetermined region of the touch panel is operated through a touch on a desired operation switch in the operation panel 21, the system controller 22 can perform later-described operational inputs for, for example, the setting of operation data of the recording target and the setting and changing of a set value, such as a threshold value.

A remote controller 30, which forms a second central operation device operated by an operating surgeon or the like located in a sterile area, enables other devices to be operated through the system controller 22, when communication is established between the remote controller 30 and the devices.

The system controller 22 is connected to a patient monitoring system 4 by a cable 9. Therefore, as described later, the system controller 22 can record biological information obtained from the patient monitoring system 4 in the HDD 22a and display the recorded biological data, such as the data of the pulse and the blood pressure, for example, on the required display device 19.

Further, the system controller 22 is equipped with an infrared communication port (not illustrated), which serves as communication means. The infrared communication port is provided at a position facilitating the irradiation of infrared light, such as the vicinity of the display device 19, and the connection to the system controller 22 is established by a cable.

In the endoscopic operation system 3 according to the above-described configuration, out of the various types of operation data, data specified, i.e., set as described later is chronologically recorded in the HDD 22a from the start to the end of an operation. That is, after the start of the operation, the system controller 22 records the specified data in the HDD 22a as the operation data, out of the biological data of the patient inputted from the patient monitoring system 4 and the similarly inputted data of operation-related devices. In the process, the operation data is recorded in association with a predetermined time data, such as the elapsed time since the start of the operation. Specifically, as well as the time data, such data as the pulse and the blood pressure is recorded in the HDD 22a as the biological data. Further, as the data of the operation-related devices, the data sent from the medical devices such as the ultrasonic operation device 13 and the data sent from peripheral devices such as a receiving device 5 and the VTR 17 are also recorded in the HDD 22a.

The time data associated with the operation data may be data indicating the elapsed time since the start of the operation, with the start time of the operation set as zero, or may be data of so-called Japan Standard Time indicating the date and time of the operation.

Further, in the present embodiment, only the operation data specified, i.e., set as described later is recorded in the HDD 22a, which serves as the recording device. However, all of the operation data may be first recorded in a first recording unit (a first storage area) within the HDD 22a, and only the set data out of all of the operation data recorded in the first recording unit may be recorded as second data in a second recording unit (a second storage area or a recording device other than the HDD 22a) within the HDD 22a.

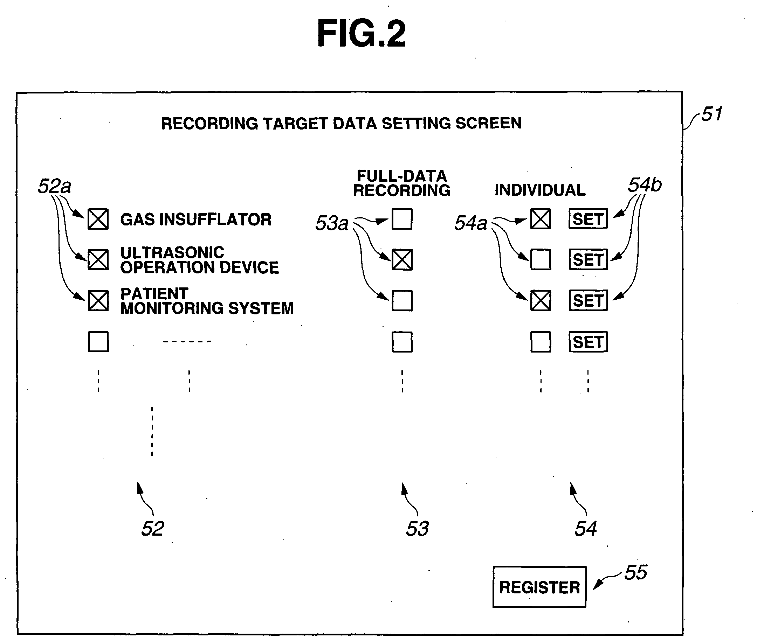

Fig. 2 is a diagram illustrating an example of a recording target data setting screen for setting the recording target. The screen of Fig. 2 is a screen displayed on the display device 19 when the system controller 22 generates screen data upon a predetermined operation on the operation panel 21 by a surgeon or the like, for example. As described below, the screen of Fig. 2 is a screen for displaying on the screen of the display device 19 a list of operation data to be inputted, and for prompting the surgeon to select operation data of the recording target from the displayed operation data.

With the use of the screen of Fig. 2, the surgeon or the like sets the recording target data. A recording target data setting screen 51 includes a list of operation data to be inputted in the system controller 22. The recording target data setting screen 51 of Fig. 2 includes a recording target device display section 52 in which the operation-related devices (specifically, the names of the devices) are displayed in a list format, a full-data recording specification section 53 in which the recording of all data of a device is specified for each of the devices, and a detail specification section 54 in which data to be recorded is set for each of the devices and the recording of the set partial data of the device is specified.

In Fig. 2, the letters representing the gas insufflator, the ultrasonic operation device, the patient monitoring system, and so forth are included in the recording target device display section 52 in the list format. For example, to specify the gas insufflator as the target device of the recording, a checkbox 52a in the recording target device display section 52 corresponding to the gas insufflator is checked through the operation of the operation panel 21 by the surgeon or the like. In Fig. 2, the respective checkboxes corresponding to the gas insufflator, the ultrasonic operation device, and the patient monitoring system are checked. Further, which part of the operation data should be recorded is set for each of the devices by checking either one of checkboxes 53a and 54a of the full-data recording specification section 53 and the detail specification section 54. In Fig. 2, the checkboxes 54a of the detail specification section 54 are checked for the gas insufflator and the patient monitoring system, while the checkbox 53a of the full-data recording specification section 53 is checked for the ultrasonic operation device. Fig. 2 therefore illustrates the setting in which all data is recorded for the ultrasonic operation device and only individually specified data is recorded for the gas insufflator and the patient monitoring system. The recording target data setting screen 51 of Fig. 2 and the processing of generating the screen data therefor form a display selection unit for prompting the surgeon to select the operation data of the recording target.

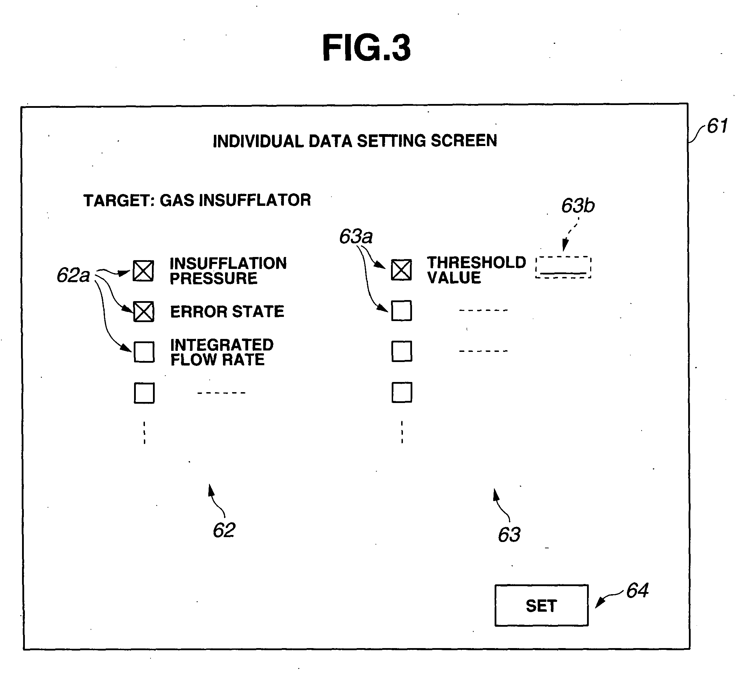

Fig. 3 is a diagram illustrating an example of an individual data setting screen for setting which part of the data of a device should be recorded. When one of the checkboxes 54a in the detail specification section 54 of Fig. 2 is checked and a corresponding detail setting button 54b in the detail specification section 54 is selected (e.g., clicked), the individual data setting screen of Fig. 3 is displayed on the screen of the display device 19.

Fig. 3 illustrates an individual data setting screen 61 for the gas insufflator. The individual data setting screen 61 includes an item display section 62 for displaying the item names of data used to set which part of the data of the gas insufflator should be recorded. Checkboxes 62a for the specification are displayed for the respective items. When one of the checkboxes 62a corresponding to the item to be recorded out of the items displayed in the list format is checked through the operation of the operation panel 21 by the surgeon or the like, the item to be recorded is set. In Fig. 3, the checkboxes 62a for the items of the insufflation pressure and the error state are checked, while the other items are unchecked. Therefore, all of the data of the gas insufflator is not recorded, but only the data of the insufflation pressure and the error state of the gas insufflator is recorded.

The individual data setting screen 61 further includes a condition setting section 63 in which the condition for the recording is set. The section includes checkboxes 63 a for specifying whether or not to set a threshold value or the like corresponding to each of the items. In Fig. 3, one of the checkboxes 63a corresponding to the item of the insufflation pressure is checked to set the threshold value. In an input field 63b in which a specific threshold value is inputted, the surgeon can input threshold value data which should be set. The threshold value forms condition data, according to which the data is recorded only when the insufflation pressure exceeds "20 mmHg," for example. The individual data setting screen 61 of Fig. 3 forms a condition setting unit for setting the condition for recording the operation data for each of the operation-related devices. Particularly, the input field 63b in which the threshold value is inputted forms a condition setting unit for setting the recording condition as a predetermined threshold value forming the basis for the comparison of the data of the device.

Subsequent to the completion of the setting of the individual data in Fig. 3, the surgeon presses (clicks) a setting button 64 for the setting to return to the screen of Fig. 2. In the case of Fig. 2, the patient monitoring system is neither set for the full-data recording. Thus, similarly to Fig. 3, an individual data setting screen for the patient monitoring system is used to set the recording target.

With the use of the recording target data setting screen and the individual data setting screen, the surgeon sets the data to be recorded out of the operation data inputted in the system controller 22. When all of the settings are completed, the surgeon presses (clicks) a registration button 55 of Fig. 2. Through the pressing of the registration button 55, the content set with the use of the recording target data setting screen and the individual data setting screen is stored, as recording target set data, in a predetermined storage area within the HDD 22a of the system controller 22.

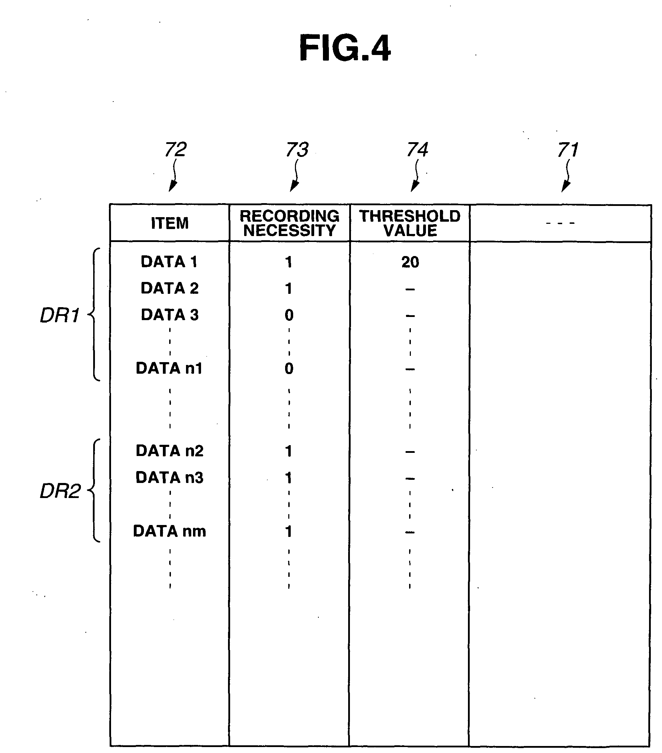

Fig. 4 is a diagram illustrating a configuration example of the recording target set data. A recording target set data table 71, which is data in a table format, includes an item field 72, a recording necessity flag field 73, and a threshold value field 74. The content set with the setting screens of Figs. 2 and 3 is written in accordance with the respective items of the recording target set data table 71.

For example, Data DR1 in the range from Data 1 to Data n1 of Fig. 4 represents the data of the gas insufflator, and "1" is written in the data of the recording necessity flag field 73 only for the data set with the individual data setting screen of Fig. 3, while "0" is written for items which have not been set to be recorded.

Further, Data DR2 in the range from Data n2 to Data nm represents the data of the ultrasonic operation device. Since the full-data recording has been selected in the recording target data setting screen of Fig. 2, "1" is written in the data of the recording necessity flag field 73 for all of the items.

The data of the content set with the use of the recording target data setting screen and the individual data setting screen is thus written in the recording target set data table 71 of Fig. 4.

Further, the threshold value data as the recording condition for the Data 1 (the insufflation pressure in the above-described example) is written in the threshold value field 74 of the Data 1. The threshold value data is the condition data inputted in the input field 63b of Fig. 3 and written in the threshold value field 74 of the recording target set data table 71 of Fig: 4. Similarly, the recording condition for another item can be set with the individual data setting screen of Fig. 3 or another screen, and is written as the recording condition in the threshold value field 74 of Fig. 4 or another field. In Fig. 4, the threshold value field 74 is a field for setting the threshold value as the condition for recording the data of the corresponding item. In Fig. 4, the threshold value is not set for the Data 2, and there is no particular condition for the data. Thus, all data of the Data 2 will be recorded.

In the present embodiment, the example is shown in which the recording condition is set through the setting of the threshold value. However, the recording condition may be set not by the threshold value but by a data range, and also by the comparison with other data, the state of the other data, and so forth.

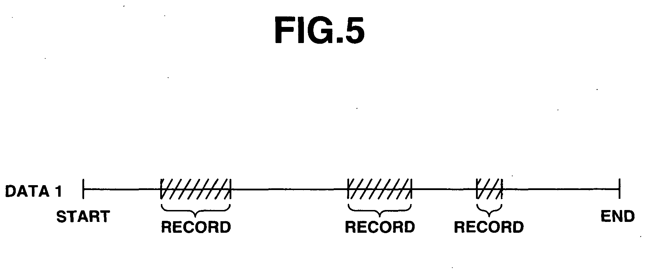

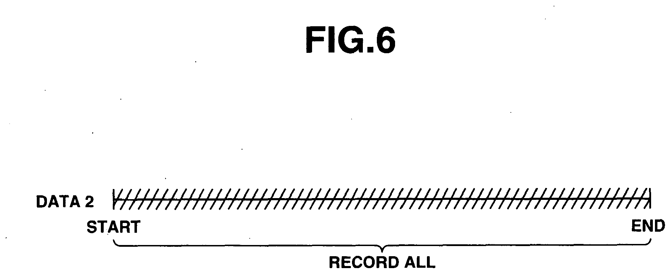

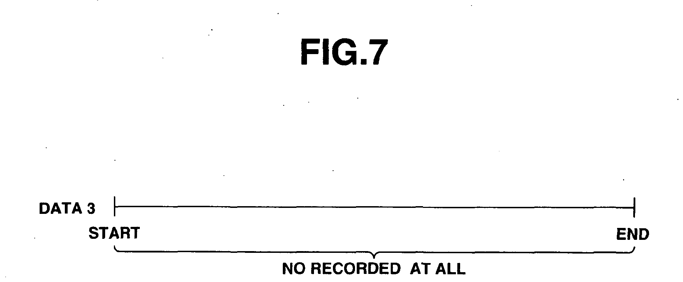

In the above-described example, therefore, the Data 1 is recorded only when the set recording condition is matched. Further, the Data 2 is all recorded, while the Data 3 is not recorded at all. Figs. 5 to 7 are diagrams illustrating recorded states during a time from the start to the end of the operation. Fig. 5 is a diagram illustrating the recorded state of the Data 1, showing that the Data 1 is recorded only when the recording condition is matched. That is, the figure illustrates that the data is recorded only when the insufflation pressure exceeds 20 mmHg in the above-described example. Fig. 6 is a diagram illustrating the recorded state of the Data 2, showing that all of the Data 2 is recorded. Fig. 7 is a diagram illustrating the recorded state of the Data 3, showing that the Data 3 is not the target of the recording and thus is not recorded at all.

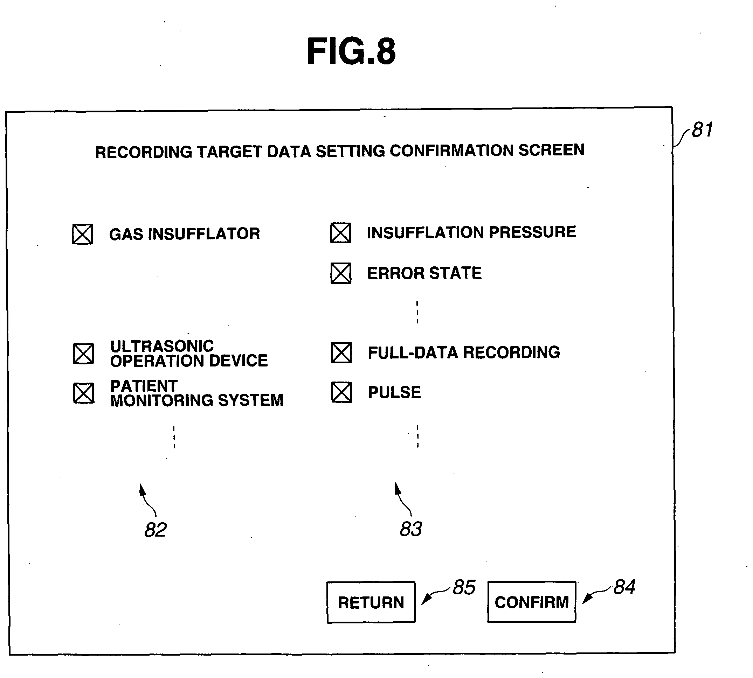

Fig. 8 is a diagram illustrating an example of a confirmation screen for confirming the set content, and is displayed on the screen of the operation panel 21 through the pressing (click) of the registration button 55 on the recording target data setting screen of Fig. 2. The surgeon can confirm the set content by viewing the confirmation screen of Fig. 8.

A recording target data setting confirmation screen 81 of Fig. 8 includes a list of operation data to be recorded. The example of Fig. 8 includes a recording target device display section 82 and a recording target display section 83 for the respective devices, both of which are displayed in a list format.

In Fig. 8, the letters representing the gas insufflator, the ultrasonic operation device, the patient monitoring system 4, and so forth are included in the recording target device display section 82 in the list format. Further, the figure shows that the insufflation pressure, the error state, and so forth are to be recorded out of the operation data corresponding to the gas insufflator included in the recording target device display section 82. As for the operation data of the ultrasonic operation device, "full-data recording" is displayed. Thus, the figure shows that all of the data of the device is to be recorded. As for the patient monitoring system, the figure shows that the pulse is to be recorded out of the operation data of the system.

When the recording condition has been set for each of the items, the condition therefor, such as the threshold value, for example, may be displayed as well in the recording target data setting confirmation screen 81 of Fig. 8.

By viewing the recording target data setting confirmation screen 81, the surgeon can confirm whether the data to be recorded is correctly set. If the set content is correct, the surgeon presses (clicks) a confirmation button 84 so that the system controller 22 stores the confirmed set content in the HDD 22a. Further, if a return button 85 is pressed (clicked), the recording target data setting screen 51 of Fig. 2 is displayed.

The above-described setting of the operation data to be recorded is thus performed prior to the start of the operation, and the system controller 22 records the operation data in accordance with the set content after the start of the operation. As a result, the data recorded in the HDD 22a of the system controller 22 does not include unnecessary data.

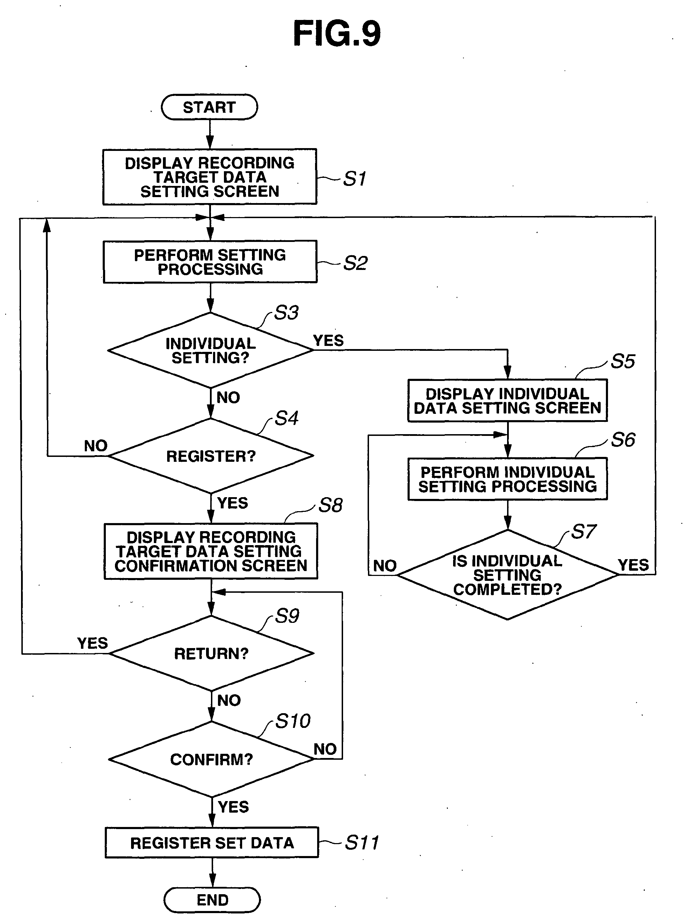

Fig. 9 is a flowchart illustrating an example of the flow of processing of setting the above-described recording target data. The surgeon first operates the operation panel 21 to display the recording target data setting screen 51 on the screen of the operation panel 21, to thereby perform the processing of Fig. 9. The processing of Fig. 9 and the recording target data setting screen 51 of Fig. 2 form a setting unit for setting the operation data to be recorded.

Firstly, the system controller 22 displays the recording target data setting screen 51 on the screen of the operation panel 21 (Step S1). Then, the system controller 22 performs setting processing through the operation on the recording target data setting screen 51 (Step S2). Specifically, the setting processing in the present embodiment is processing to, for example, change the display on the screen in accordance with the operation of checking a checkbox on the recording target data setting screen 51, for example.

While performing the setting processing (Step S2), the system controller 22 constantly determines whether or not the individual setting has been instructed on the recording target data setting screen 51 (Step S3). If there is no instruction for the individual setting, the system controller 22 determines whether or not the registration button 55 has been pressed (Step S4). If there is no pressing of the registration button 55, NO is determined in Step S4, and the processing of the system controller 22 returns to Step S2.

If the individual setting is instructed on the recording target data setting screen 51, YES is determined in Step S3, and the system controller 22 displays the individual data setting screen 61 of a specified device (Step S5). Then, individual setting processing is performed (Step S6). Specifically, the individual setting processing in the present embodiment is the processing to, for example, change the display on the screen in accordance with the operation of checking a checkbox on the individual data setting screen 61, for example.

While performing the individual setting processing (Step S6), the system controller 22 determines whether or not the setting button 64 has been pressed on the individual data setting screen 61 (Step S7). If there is no pressing of the setting button 64, NO is determined in Step S7, and the processing of the system controller 22 returns to Step S6.

If the setting button 64 is pressed on the individual data setting screen 61, YES is determined in Step S7, and the processing returns to Step S2 to perform the setting processing on the recording target data setting screen 51. If the registration button 55 is pressed during the performance of the setting processing, YES is determined in Step S4, and the system controller 22 displays the recording target data setting confirmation screen 81 (Step S8).

It is determined whether or not the return button 85 has been pressed on the recording target data setting confirmation screen 81 (Step S9). If the return button 85 is pressed, YES is determined in Step S9, and the processing returns to Step S2 to perform the setting processing on the recording target data setting screen 51.

It is determined whether or not the confirmation button 84 has been pressed on the recording target data setting confirmation screen 81 (Step S10), and the processing of the system controller 22 returns to Step S9 if there is no pressing of the confirmation button 84. If the confirmation button 84 is pressed, YES is determined in Step S10, and the system controller 22 registers, i.e., stores the set data of the confirmed content in the recording target set data table 71 of a predetermined storage area in the HDD 22a (Step S11).

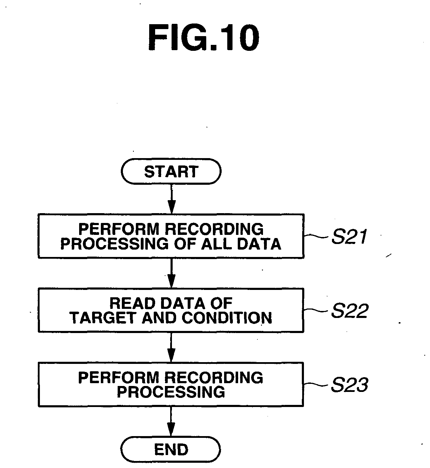

When the operation data to be recorded and the recording conditions have been thus set, the system controller 22 performs recording processing according to the setting content after the start of the operation. Fig. 10 is a flowchart illustrating an example of the flow of the recording processing of the operation data. Upon the operation of starting the recording or the input of an instruction for starting the recording, the system controller 22 performs the processing of Fig. 10.

The system controller 22 first performs recording processing of recording all of the inputted operation data (Step S21). Then, the system controller 22 reads the data of the recording target and the condition set through the processing of Fig. 9 (Step S22). The system controller 22 performs recording processing on the basis of the read set content (Step S23). In the recording processing, the operation data of the set recording target, and the matched operation data if the recording condition has been set, are recorded in the HDD 22a in association with a predetermined time data. The processing of Steps S21 to S23 may be performed and repeated every time each of the operation data is inputted. Alternatively, after all of the data or a part of the operation data of a certain amount has been inputted, the data may be subjected to the processing of Step S21 and then to the processing of Steps S22 and S23. In the above process, Step S21 of Fig. 10 forms an all data recording unit for recording all of the data. Further, Steps S22 and S23 of Fig. 10 form an operation data recording unit for recording only the target data to be recorded.

As described above, the system controller 22, which forms the operation data management device according to the present embodiment, records only the set, i.e., specified operation data in the HDD 22a. Therefore, the system controller 22 does not record unnecessary operation data. Consequently, the surgeon can easily check the content of the operation.

The above-described example is an example relating to the recording of the operation data, in which the data to be recorded is performed is set, i.e., specified. Similarly, data to be displayed and data to be printed can be specified, i.e., set in the display and the printing of the operation data.

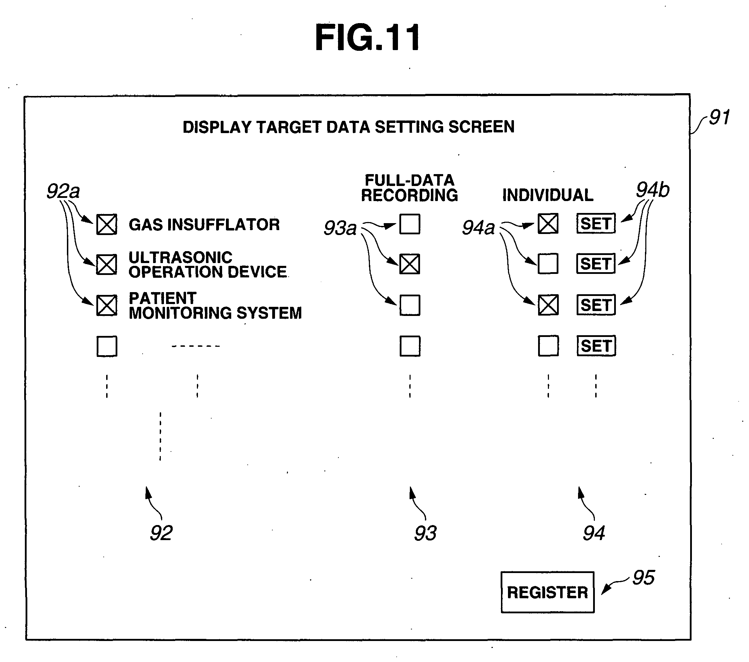

Fig. 11 is a diagram illustrating an example of a display target data setting screen for setting a display target. Similarly to the above-described recording target data setting screen, the screen of Fig. 11 is also a screen displayed on the display device 19 when the system controller 22 generates screen data upon a predetermined operation on the operation panel 21 by the surgeon or the like, for example.

With the use of the screen of Fig. 11, the surgeon or the like sets display target data. A display target data setting screen 91 includes a list of operation data to be inputted in the system controller 22. The display target data setting screen 91, which is similar in configuration to the recording target data setting screen 51, includes a display target device display section 92 in which the operation-related devices are displayed in a list format, a full-data display specification section 93 in which the display of all data of a device is specified for each of the devices, and a detail specification section 94 in which data to be displayed is set for each of the devices and the display of the set partial data of the device is specified.

Further, an individual data setting screen similar to the individual data setting screen 61 of Fig. 3 can be displayed to set which part of the data of a device should be displayed. When one of checkboxes 94a in the detail specification section 94 of Fig. 11 is checked and a corresponding detail setting button 94b in the detail specification section 94 is selected (e.g., clicked), the individual data setting screen is displayed on the screen of the display device 19. Since the individual data setting screen is the same as the screen of Fig. 3, description thereof will be omitted herein.

Accordingly, the data to be displayed can also be set with the use of the display target data setting screen 91 of Fig. 11 and the individual data setting screen (not illustrated). Therefore, the system controller 22, which forms the operation data management device according to the present embodiment, displays only the set, i.e., specified operation data on the display device 19, and thus does not display unnecessary operation data. Consequently, the surgeon can easily check the content of the operation.

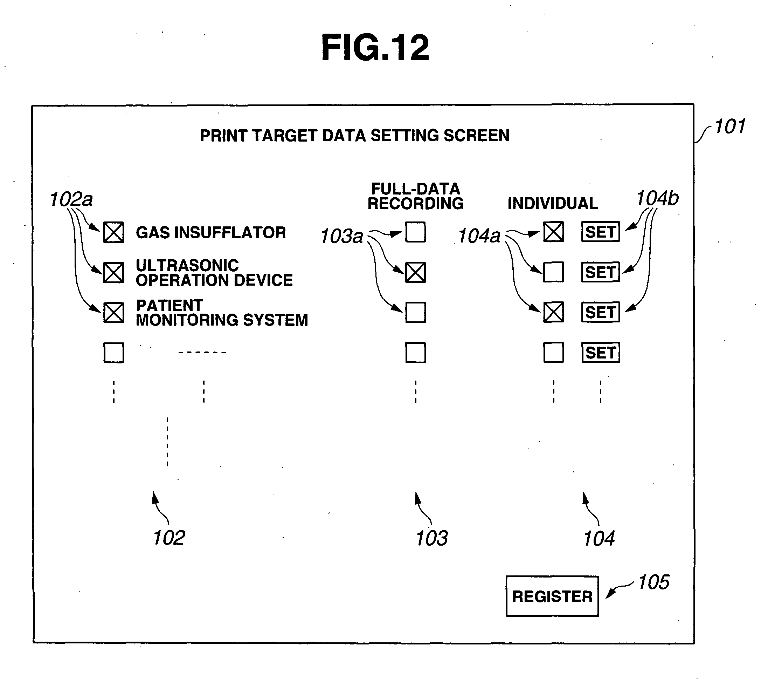

Fig. 12 is a diagram illustrating an example of a print target data setting screen for setting a print target. Similarly to the recording target data setting screen and the display target data setting screen described above, the screen of Fig. 12 is also a screen displayed on the display device 19 when the system controller 22 generates screen data upon a predetermined operation on the operation panel 21 by the surgeon or the like, for example.

With the use of the screen of Fig. 12, the surgeon or the like sets print target data. A print target data setting screen 101 includes a list of operation data to be inputted in the system controller 22. The print target data setting screen 101, which is also similar in configuration to the recording target data setting screen 51, includes a print target device display section 102 in which the operation-related devices are displayed in a list format, a full-data print specification section 103 in which the printing of all data of a device is specified for each of the devices, and a detail specification section 104 in which data to be printed is set for each of the devices and the printing of the set partial data of the device is specified.

Further, an individual data setting screen similar to the individual data setting screen 61 of Fig. 3 can be displayed to set which part of the data of a device should be printed. When one of checkboxes 104a in the detail specification section 104 of Fig. 12 is checked and a corresponding detail setting button 104b in the detail specification section 104 is selected (e.g., clicked), the individual data setting screen is displayed on the screen of the display device 19. The individual data setting screen is the same as the screen of Fig. 3, and thus description thereof will be omitted herein.

Accordingly, the data to be printed can also be set with the use of the print target data setting screen 101 of Fig. 12 and the individual data setting screen (not illustrated). Therefore, the system controller 22, which forms the operation data management device according to the present embodiment, prints only the set, i.e., specified operation data on paper through a printing device, and thus does not print unnecessary operation data. Consequently, the surgeon can easily check the content of the operation.

Conventionally, when necessary data is displayed and checked out of the recorded operation data, it is inefficient to display data which will not need to be checked. Similarly, when necessary data is printed and checked out of the recorded operation data, it is inefficient to print data which will not need to be checked.

In particular, the recording or the display, for example, of the operation data which will not need to be checked entails a problem of taking a long time to check the content of the operation. Particularly, if the operation time is long, there is a problem of taking a long time to postoperatively analyze the recorded operation data and assess the content of the operation.

According to the above-described embodiment, however, inefficient display or recording of the operation data is not performed. Therefore, the content of the operation can be easily checked.

As described above, in the recording of the operation data, all of the operation data may be recorded as the first record data, and when the data for checking the content of the operation is recorded as the second record data, only the operation data of the set recording target may be recorded.

Similarly, also in the display and the printing of the operation data, processing corresponding to Steps S22 and S23 of Figs. 10 may be performed so that, when data is displayed or printed out of the recorded first record data (all of the operation data), the operation data of the display target or print target set with the use of Fig. 11 or 12 is extracted to display only the operation data of the set display target or print target. Alternatively, the operation data of the similarly set display target or print target may be extracted from the second record data, in which only the recording target set as the data for checking the content of the operation is recorded, so that only the operation data of the set display target or print target is displayed.

In the above-described example, the system controller 22, which forms the operation data management device, performs the display or print setting processing and the display or print processing. However, a computer other than the system controller 22 may perform the display or print setting processing and the display or print processing described above.

Accordingly, in the recording, the display, and the printing described above, the recording target, the display target, and the print target may be respectively set individually and separately. Alternatively, each of the display target and the print target may be set in association with the operation data of the set recording target so as to be extracted from the operation data.

With the system controller 22 thus configured to be able to perform the setting of the data of the display target and the print target, in addition to the setting of the data of the recording target, the target data can be selected in the recording, the display, and the printing. Therefore, the recording of unnecessary data is prevented. As a result, the surgeon can easily perform a postoperative check of the content of the operation.

As described above, as well as the control of the medical devices such as the endoscopic camera device 15, the recording, the display, and the printing of the data of the medical devices are performed by the system controller 22. Specific examples of the recording and the display will be described below.

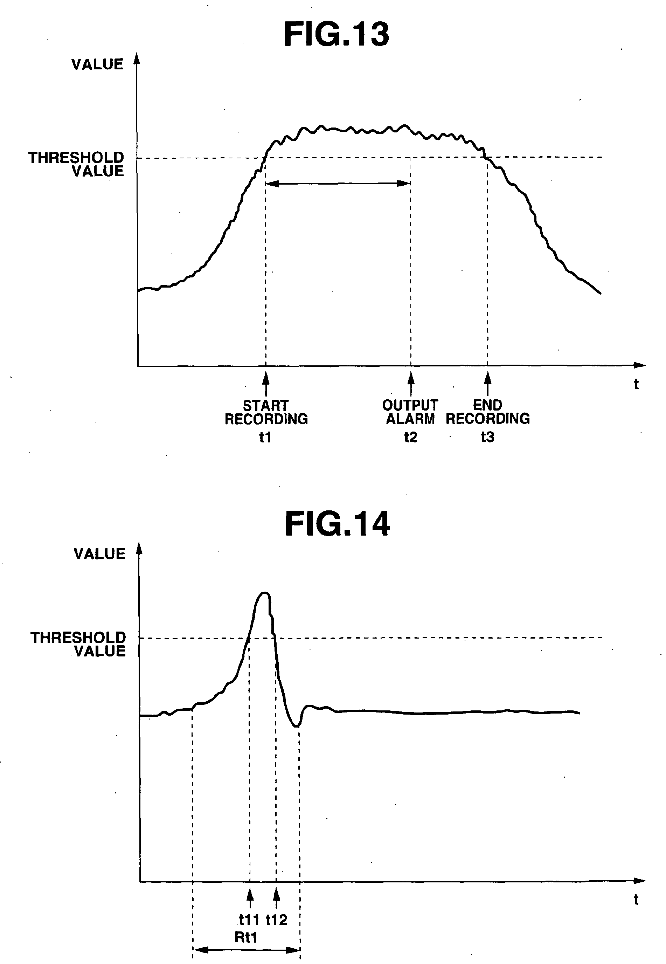

Figs. 13 and 14 are graphs for explaining which range of data is recorded when a predetermined threshold value has been set as the recording condition. Fig. 13 is a graph illustrating how certain operation data has changed in accordance with the elapse of time. A predetermined threshold value has been set, and the system controller 22 starts recording the operation data when the operation data exceeds the threshold value at a time t1, and then ends recording the operation data when the operation data falls below the threshold value at a time t3. Further, the system controller 22 includes an alarm output unit for outputting alarm when the recording condition is satisfied and the elapsed time since the start of the recording of the operation data exceeds a predetermined time threshold value. Therefore, even if the system controller 22 is configured to output alarm upon passage of a predetermined time t2 since the excess of the operation data over the threshold value, the system controller 22 records the data during the time in which the operation data exceeds the threshold value.

The data recorded in the above case may not be the data obtained since the excess of the data over the threshold value until the fall of the data below the threshold value. For example, the system controller 22 may be configured such that, when the recording condition is satisfied, the system controller 22 performs the recording of the operation data since a time earlier than the time point of the satisfaction of the recording condition by a predetermined time. In such a case, if the system controller 22 is configured to always store the operation data temporarily in a memory for a predetermined time, and when the operation data exceeds the threshold value, as illustrated in Fig. 14, the system controller 22 can record the operation data of a period (Rt1) since a time earlier by a predetermined time than a time point t11 of the excess of the operation data over the threshold value to a time later by a predetermined time than a time point t12 of the fall of the operation data below the threshold value.

Although the description has been made of the data recording in the examples of Figs. 13 and 14, the description similarly applies to the display or the printing.

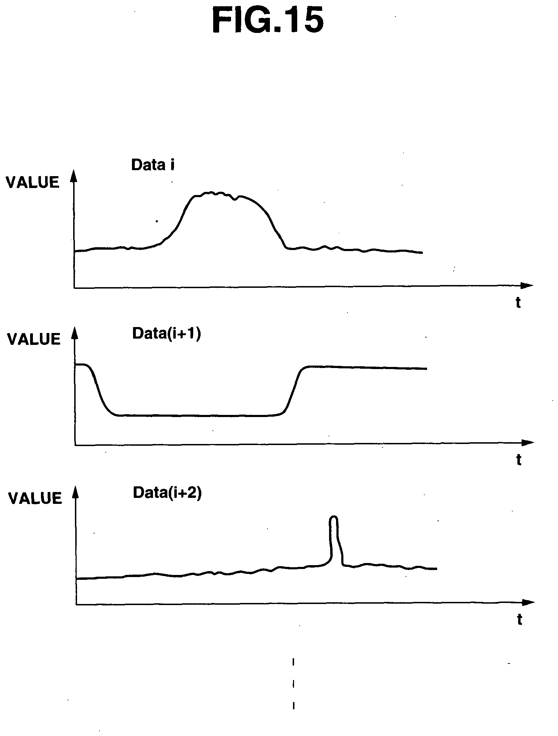

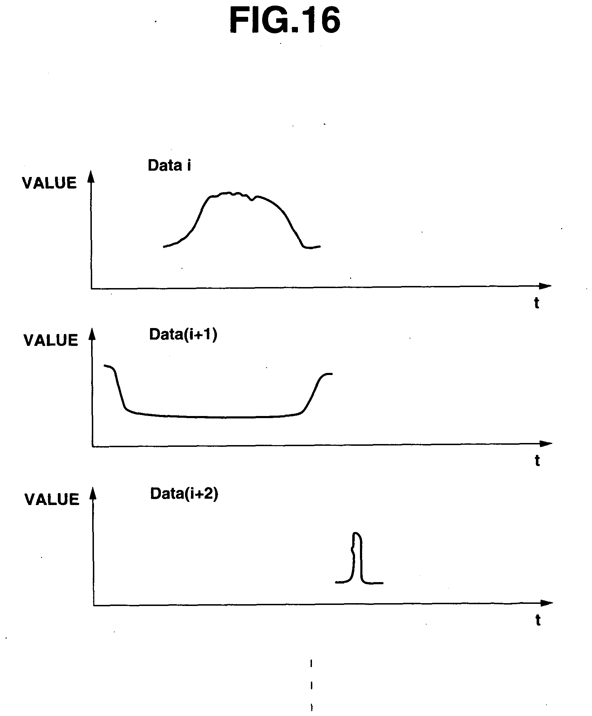

Further, if the data display condition is set to display a data portion of certain operation data, in which the operation data has changed by equal to or greater than a predetermined value, the operation data having such a change can be displayed, being aligned in terms of the time axis, as illustrated in Figs. 15 and 16. Fig. 15 is graphs illustrating an example in which three or more operation data Data i, Data (i+1), Data (i+2), and so forth are displayed on the screen of the display device 19, being aligned in terms of the time axis.

In the display, the display may be limited only to a data portion of the respective operation data since the excess of the operation data over the threshold values thereof (or since before the excess) until the fall of the operation data below the threshold values thereof (or until after the fall). Fig. 16 is a diagram illustrating an example in which the display is limited only to a data portion since the excess of the operation data over the threshold value until the fall of the operation data below the threshold value, and a data portion since the fall of the operation data below the threshold value until the excess of the operation data over the threshold value.

Although the description has been made of the data display in the examples of Figs. 15 and 16, the description similarly applies to the recording or the printing.

That is, only the portions indicated by the solid lines in Figs. 15 and 16 may be recorded or printed.

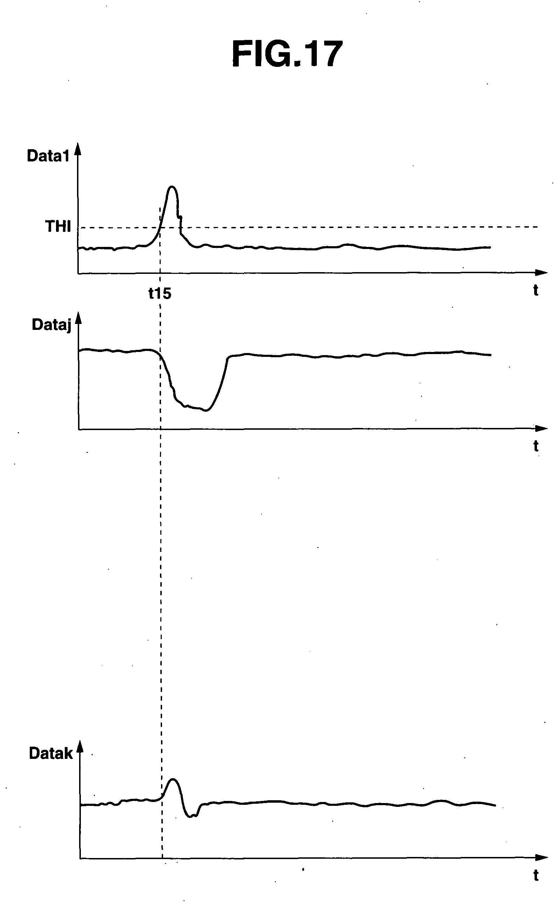

Fig. 17 is graphs illustrating an example in which the display condition is set to display only data showing a simultaneously change out of the operation data. As illustrated in Fig. 17, Data 1 has changed at a time point t15, and Data j and Data k have simultaneously changed rapidly at the substantially same time point. Therefore, the condition as described above may be set as the display condition.

Although the description has been made of the data display in the example of Fig. 17, the description similarly applies to the recording or the printing.

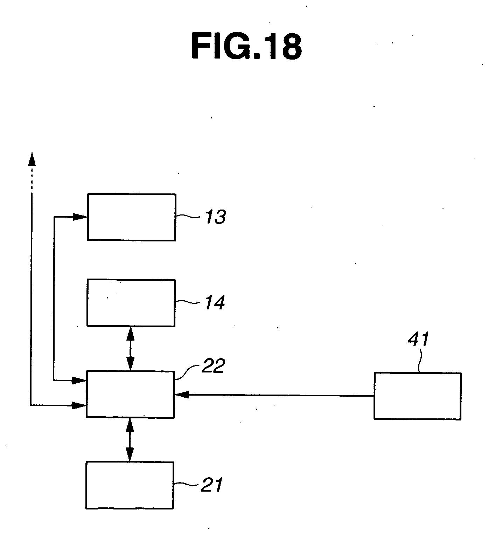

Further, as described above, the system controller 22 is connected to the variety of medical devices to be capable of performing the data communication with the variety of medical devices and controlling the devices. However, there is a case in which the system controller 22 is also connected to a medical device, on which the system controller 22 does not perform the control, but with which the system controller 22 only performs the data communication. Fig. 18 is a system configuration diagram illustrating an example in which the system controller 22 is also connected to a medical device, on which the system controller 22 does not perform the control, but with which the system controller 22 performs the data communication. In Fig. 18, the same constituents as the constituents of Fig. 1 are assigned with the same reference numerals, and description thereof will be omitted. Fig. 18 is different from Fig. 1 in that, in addition to the configuration of Fig. 1, an anesthesia device 41 is connected to the system controller 22 as the medical device with which the system controller 22 only performs the data communication. In Fig. 18, the illustration of other medical devices unrelated to the following description is omitted.

In an endoscopic surgical operation, the anesthesia device 41 is employed as well as the ultrasonic operation device 13 and the gas insufflator 14. During the endoscopic surgical operation, carbon dioxide gas is supplied into a peritoneal cavity of a patient with the use of the gas insufflator 14 to ensure the workspace and the field of view for the surgeon.

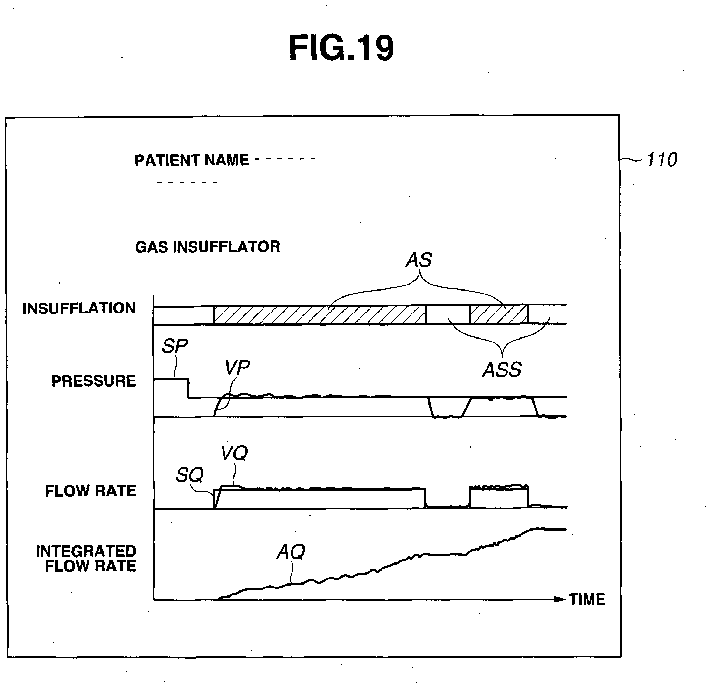

Fig. 19 is a diagram illustrating an example of graphs displaying, as the operation data of the gas insufflator 14, the performance data of the gas insufflator 14 recorded in association with a predetermined time. The diagram of Fig. 19 is displayed on the screen of the display device 19, for example.

The gas insufflator 14 starts and stops the insufflation of carbon dioxide gas under a predetermined instruction. In Fig. 19, hatched portions indicate an insufflation state AS, while unhatched portions indicate a state ASS in which the insufflation is stopped. Therefore, with the horizontal axis representing the time axis, the insufflation period and the insufflation stop period can be recognized at a glance along the elapse of a predetermined time.

Along the same time axis, a set pressure SP and an actual pressure VP are graphically displayed. Similarly, a set flow rate SQ and an actual flow rate VQ are graphically displayed along the same time axis. Further, an integrated flow rate AQ since the start of the insufflation is similarly graphically displayed.

The system controller 22 detects a decrease in the amount of carbon dioxide gas insufflated from the gas insufflator 14 or the stop of the insufflation. The system controller 22 may be configured to, upon detection of the decrease or the stop of the gas insufflator 14, control the recording so as to change the time interval of the recording time to increase or decrease the recording interval of the data, for example, or so as to stop the recording operation. For example, the recording interval is 0.1 seconds during the insufflation of carbon dioxide gas by the gas insufflator 14, but is changed to 5 seconds upon detection of the decrease or the stop of the insufflation of carbon dioxide gas by the gas insufflator 14.

Further, if there is another device relating to the performance of the gas insufflator 14, the performance of the another device may be recorded, for example, in accordance with the performance of the gas insufflator 14. For example, there is a case in which the another device is not used during the period in which the gas insufflator 14 performs the insufflation (the period of AS) but is used during the period in which the insufflation is stopped (the period of ASS) in Fig. 19. In such a case, the system controller 22 may be configured to, upon detection of the stop of the insufflation by the gas insufflator 14, record the performance information, the inputted information, the set information, and so forth of the another device during the period (the period of ASS). That is, on the basis of the performance data, which is the operation data received from the gas insufflator 14 as a predetermined device, the system controller 22 changes the operation data set to be recorded and records the operation data of the predetermined another device.

Further, the system controller 22 may change the recording interval of the performance data of each of the devices in accordance with the detected value of the device connected to the system controller 22. For example, the system controller 22 may be configured to control the recording so as to reduce the recording interval of the performance data from 5 seconds to 0.1 seconds, for example, to save a detailed record, if an abnormal value, a large increase or decrease, or the like is found in the detected value obtained through the detection of the biological information, such as the oxygen saturation by pulse oximetry (SPO2) of the anesthesia device 41 and the pulse rate of a patient controlled by the anesthesia device 41.

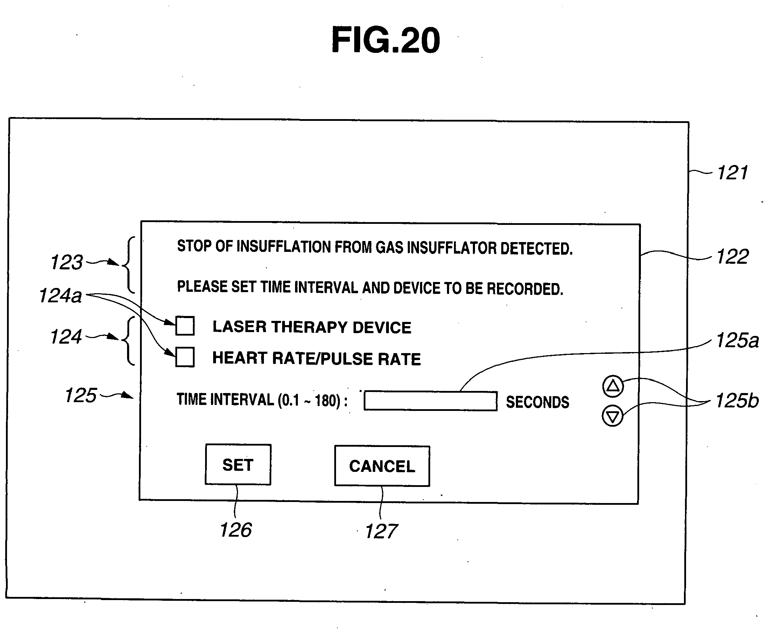

Further, the system controller 22 may be configured to display the screen illustrated in Fig. 20 on the display device 19 upon detection of the decrease in the amount of carbon dioxide gas insufflated from the gas insufflator 14 or the stop of the insufflation so that the time interval of the recording time can be changed. Fig. 20 is a diagram illustrating an example of a setting screen for changing the time interval of the recording time.

Upon detection of the stop of the insufflation of carbon dioxide gas by the gas insufflator 14 and so forth, the system controller 22 displays the screen of Fig. 20 on the display device 19. A screen 122 of Fig. 20 is displayed within a screen 121 of the display device 19 in the form of a pop-up window. The screen 122 includes a specification content description section 123 which notifies that the specification of a target device having data to be recorded and the setting of the time interval can be performed, a target device specification section 124 in which the target device is specified, and a time interval setting section 125 in which the time interval is set.

The specification content description section 123 is a message display section for describing the reason for the display of the screen of Fig. 20 and the content which can be set.

The target device specification section 124 is a section for displaying a device which can be set in the time interval of the recording time when the gas insufflator 14 is brought into a predetermined state, to thereby prompt a user to perform the setting. In the present example, a laser therapy device, the heart rate, and the pulse rate can be recorded. The device to be recorded or the operation data to be recorded can be specified by checking a corresponding checkbox 124a displayed in the vicinity of the device desired to be recorded.

The time interval setting section 125 includes a value input field 125a in which the value of the time interval of the recording time is set. The user can set the value of the time interval by inputting the value in the value input field 125a. Further, the user can increase or decrease the value displayed in the value input field 125a by selecting either one of an increase button and a decrease button 125b provided in the vicinity of the value input field 125a.

The user presses a setting button 126 to register in the system controller 22 the content set or specified on the screen 121, and presses a cancel button 127 to cancel the setting and so forth.

The system controller 22 is configured to include a central processing unit (CPU) and to execute a predetermined program. Therefore, the CPU included in the system controller 22, the processing of generating screen data for displaying the screen of Fig. 20 on the display device 19, and the processing of changing the time interval of the recording time in accordance with the content set on the screen form a time interval changing unit.

Further, the performance information, the inputted information, the set information, and so forth may be individually recorded in each of the medical devices, which are controlled devices. For example, in the case of the anesthesia device 41 of Fig. 18, in addition to the anesthesia record information including the type, the time, and the amount of anesthesia and the information of the oxygen saturation by pulse oximetry (SPO2), the inputted patient information, the biological information, the set information for controlling the amount of anesthesia, and so forth may be individually stored in a not-illustrated recording device of the device (i.e., a recording device of the anesthesia device 41 forming a medical device). In such a case, too, in accordance with the insufflation state of carbon dioxide gas from the gas insufflator 14, e.g., in accordance with the detection of the decrease in the insufflated amount or the stop of the insufflation, the system controller 22 receives from the anesthesia device 41 the value of the oxygen saturation by pulse oximetry (SPO2) in the insufflation of carbon dioxide gas at a predetermined time interval (e.g., every second), and stores and manages only necessary information in a storage device of the system controller 22. A treatment is directly performed on tissue or an organ at the output timing from the electric scalpel device 13. Thus, the system controller 22 may be configured to obtain the biological information from the anesthesia device 41 in accordance with the output timing, and to store and manage the information in the storage device.

Accordingly, in receiving the operation data of each of the plurality of medical devices from the device through communication, the system controller 22 may be configured to receive and record the operation data stored in the recording device of each of the medical devices.

As described above, according to the operation data management device of the present embodiment, the recording of unnecessary operation data is not performed. Thus, the surgeon can easily check the content of the operation. Further, the display and the printing of unnecessary operation data can be similarly prevented. Thus, the surgeon can easily check the content of the operation.

The present invention is not limited to the above-described embodiment. Thus, various modifications, alternations, and so forth can be made within a scope without changing the gist of the present invention.

## Claims

1. An operation data management device (22), comprising:
a setting unit (51, 61, 81, 91) configured for setting operation data to be recorded out of operation data from a plurality of medical devices including a gas insufflator (14); and
an operation data recording unit (S22, S23) configured for recording the operation data set by the setting unit (51, 61, 81, 91) in a recording device in association with a predetermine time,
**characterized in that** the operation data recording unit (S22, S23) is configured to:
change the operation data set to be recorded of another predetermined medical device based on the performance of the gas insufflator (14) which is received from the gas insufflator (14), and
record the operation data of the another predetermined medical device which is different from the gas insufflator (14) based on the performance data of the gas insufflator (14).

2. The operation data management device according to Claim 1, wherein the setting unit includes a display selection unit configured for causing a display device (19) to display the operation data so that operation data to be a recording target is selected from the displayed operation data, and wherein the operation data to be recorded is set by the display selection unit.

3. The operation data management device according to Claim 2, wherein the display selection unit includes a recording target device display section configured to display operation-related devices, a full-data recording specification section configured such that the recording of all data is specified for each of the operation-related devices, and a detail specification section configured such that data to be recorded is specified for each of the operation-related devices.

4. The operation data management device according to Claim 3, wherein the display selection unit further includes a condition setting section (61) configured such that a recording condition for recording the operation data is set for each of the operation-related devices.

5. The operation data management device according to Claim 4, wherein the recording condition is defined as a result of comparison between the operation data and a predetermined threshold value.

6. The operation data management device according to Claim 5, comprising an alarm output unit configured for outputting alarm when the recording condition is satisfied and the elapsed time since the start of the recording of the operation data by the operation data recording unit exceeds a predetermined time threshold value.

7. The operation data management device according to Claim 5, configured such that, when the recording condition is satisfied, the operation data recording unit performs the recording of the operation data since a time earlier than the time point of the satisfaction of the recording condition by a predetermine time.

## Patentansprüche

1. Anwendungsdatenverwaltungsgerät (22), umfassend:
eine Einstelleinheit (51, 61, 81, 91), die zum Einstellen von aufzuzeichnenden Anwendungsdaten aus Anwendungsdaten von einer Vielzahl von medizinischen Geräten ausgebildet ist und einen Gasinsufflator (14) beinhaltet; und
eine Anwendungsdatenaufzeichnungseinheit (S22, S23), die zum Aufzeichnen der durch die Einstelleinheit (51, 61, 81, 91) eingestellten Anwendungsdaten in einem Aufzeichnungsgerät im Zusammenhang mit einer vorbestimmten Zeit ausgebildet ist,
**dadurch gekennzeichnet, dass** die Anwendungsdatenaufzeichnungseinheit (S22, S23) ausgebildet ist zum:
Ändern der zum Aufzeichnen eingestellten Anwendungsdaten eines anderen vorbestimmten medizinischen Geräts basierend auf der Leistung des Gasinsufflators (14), die von dem Gasinsufflator (14) empfangen wird, und
Aufzeichnen der Anwendungsdaten des anderen vorbestimmten medizinischen Geräts, die verschieden von dem Gasinsufflator (14) sind, basierend auf den Leistungsdaten des Gasinsufflators (14).

2. Anwendungsdatenverwaltungsgerät nach Anspruch 1, wobei die Einstelleinheit eine Anzeigeauswahleinheit beinhaltet, die zum Veranlassen eines Anzeigegeräts (19) zum Anzeigen der Anwendungsdaten ausgebildet ist, sodass die Anwendungsdaten, die ein Aufzeichnungsziel sind, von den angezeigten Anwendungsdaten ausgewählt werden, und wobei die aufzuzeichnenden Anwendungsdaten durch die Anzeigeauswahleinheit eingestellt sind.

3. Anwendungsdatenverwaltungsgerät nach Anspruch 2, wobei die Anzeigeauswahleinheit einen Aufzeichnungszielgerätanzeigebereich, der zum Anzeigen von anwendungsbezogenen Geräten ausgebildet ist, einen Gesamtdatenaufzeichnungsspezifizierungsbereich, der so ausgebildet ist, dass das Aufzeichnen aller Daten für jedes der anwendungsbezogenen Geräte spezifiziert wird, und einen Detailspezifikationsbereich beinhaltet, der so ausgebildet ist, dass aufzuzeichnende Daten für jedes der anwendungsbezogenen Geräte spezifiziert sind.

4. Anwendungsdatenverwaltungsgerät nach Anspruch 3, wobei die Anzeigeauswahleinheit weiter einen Bedingungseinstellbereich (61) beinhaltet, der so ausgebildet ist, dass eine Aufzeichnungsbedingung zum Aufzeichnen der Anwendungsdaten für jedes der anwendungsbezogenen Geräte eingestellt ist.

5. Anwendungsdatenverwaltungsgerät nach Anspruch 4, wobei die Aufzeichnungsbedingung als ein Ergebnis eines Vergleichs zwischen den Anwendungsdaten und einem vorbestimmten Schwellenwert definiert ist.

6. Anwendungsdatenverwaltungsgerät nach Anspruch 5, umfassend eine Alarmausgabeeinheit, die zum Ausgeben eines Alarms ausgebildet ist, wenn die Aufzeichnungsbedingung erfüllt ist und die verstrichene Zeit seit dem Start des Aufzeichnens der Anwendungsdaten durch die Anwendungsdatenaufzeichnungseinheit einen vorbestimmten Zeitschwellenwert überschreitet.

7. Anwendungsdatenverwaltungsgerät nach Anspruch 5, derart ausgebildet, dass, wenn die Aufzeichnungsbedingung erfüllt ist, die Anwendungsdatenaufzeichnungseinheit das Aufzeichnen der Anwendungsdaten um eine vorbestimmte Zeit seit einer Zeit ausführt, die früher als der Zeitpunkt des Erfüllens der Aufzeichnungsbedingung ist.

## Revendications

1. Dispositif (22) de gestion de données opérationnelles, comprenant :
une unité (51, 61, 81, 91) de définition configurée pour définir des données opérationnelles devant être enregistrées parmi des données opérationnelles provenant d'une pluralité de dispositifs médicaux incluant un insufflateur (14) de gaz ; et
une unité (S22, S23) d'enregistrement de données opérationnelles configurée pour enregistrer les données opérationnelles définies par l'unité (51, 61, 81, 91) de définition dans un dispositif d'enregistrement en association avec une durée prédéterminée,
**caractérisé en ce que** l'unité (S22, S23) d'enregistrement de données opérationnelles est configurée pour :
changer les données opérationnelles définies pour être enregistrées d'un autre dispositif médical prédéterminé sur la base de la performance de l'insufflateur (14) de gaz qui est reçue de l'insufflateur (14) de gaz, et
enregistrer les données opérationnelles de l'autre dispositif médical prédéterminé qui est différent de l'insufflateur (14) de gaz sur la base des données de performance de l'insufflateur (14) de gaz.

2. Dispositif de gestion de données opérationnelles selon la revendication 1, dans lequel l'unité de définition inclut une unité de sélection d'affichage configurée pour faire en sorte qu'un dispositif (19) d'affichage affiche les données opérationnelles de telle sorte que les données opérationnelles destinées à être une cible d'enregistrement sont sélectionnées parmi les données opérationnelles affichées, et dans lequel les données opérationnelles devant être enregistrées sont définies par l'unité de sélection d'affichage.

3. Dispositif de gestion de données opérationnelles selon la revendication 2, dans lequel l'unité de sélection d'affichage inclut une section d'affichage de dispositif cible d'enregistrement configurée pour afficher des dispositifs se rapportant aux opérations, une section de spécification d'enregistrement de données complètes configurée de telle manière que l'enregistrement de toutes les données est spécifié pour chacun des dispositifs se rapportant aux opérations, et une section de spécification de détails configurée de telle manière que les données devant être enregistrées sont spécifiées pour chacun des dispositifs se rapportant aux opérations.

4. Dispositif de gestion de données opérationnelles selon la revendication 3, dans lequel l'unité de sélection d'affichage inclut en outre une section (61) de définition de condition configurée de telle manière qu'une condition d'enregistrement pour enregistrer les données opérationnelles est définie pour chacun des dispositifs se rapportant aux opérations.

5. Dispositif de gestion de données opérationnelles selon la revendication 4, dans lequel la condition d'enregistrement est définie comme un résultat de comparaison entre les données opérationnelles et une valeur de seuil prédéterminée.

6. Dispositif de gestion de données opérationnelles selon la revendication 5, comprenant une unité de sortie d'alarme configurée pour délivrer en sortie une alarme lorsque la condition d'enregistrement est satisfaite et que la durée écoulée depuis le début de l'enregistrement des données opérationnelles par l'unité d'enregistrement de données opérationnelles excède une valeur prédéterminée de seuil de durée.

7. Dispositif de gestion de données opérationnelles selon la revendication 5, configuré de telle manière que, lorsque la condition d'enregistrement est satisfaite, l'unité d'enregistrement de données opérationnelles exécute l'enregistrement des données opérationnelles à partir d'un moment antérieur au point dans le temps de la satisfaction de la condition d'enregistrement d'une durée prédéterminée.
